(19) Europäisches Patentamt
European Patent Office
Office européen des brevets

(11) **EP 1 399 168 B1**

(12) **EUROPÄISCHE PATENTSCHRIFT**

(45) Veröffentlichungstag und Bekanntmachung des Hinweises auf die Patenterteilung:
**05.03.2008 Patentblatt 2008/10**

(21) Anmeldenummer: **02751076.7**

(22) Anmeldetag: **26.06.2002**

(51) Int Cl.:
*A61K 31/70* (2006.01)　*A61K 35/12* (2006.01)
*C12N 5/06* (2006.01)　*A61P 35/00* (2006.01)

(86) Internationale Anmeldenummer:
**PCT/EP2002/007071**

(87) Internationale Veröffentlichungsnummer:
**WO 2003/004037 (16.01.2003 Gazette 2003/03)**

(54) **XENOGENE OLIGO- ODER/UND POLYRIBONUKLEOTIDE ALS MITTEL ZUR BEHANDLUNG VON MALIGNEN TUMOREN**

XENOGENIC OLIGOOR/AND POLYRIBONUCLEOTIDES AS AGENTS FOR THE TREATMENT OF MALIGNANT TUMOURS

OLIGO- OU/ET POLYRIBONUCLEOTIDES XENOGENES UTILISES COMME AGENTS DE TRAITEMENT DE TUMEURS MALIGNES

(84) Benannte Vertragsstaaten:
**AT BE CH CY DE DK ES FI FR GB GR IE IT LI LU MC NL PT SE TR**

(30) Priorität: **28.06.2001 DE 10131148**

(43) Veröffentlichungstag der Anmeldung:
**24.03.2004 Patentblatt 2004/13**

(73) Patentinhaber: **I.P.L. International Pharmaceutics Ltd**
**52522 Ramat Gan (IL)**

(72) Erfinder: **Meisriemler, Barbara.**
**A-4112 St.Gotthard (AT)**

(74) Vertreter: **Haselhorst, Doerte**
**Lierstrasse 12 b**
**80639 München (DE)**

(56) Entgegenhaltungen:
WO-A-94/21808　　　WO-A-98/05769
WO-A-99/07394　　　DE-A- 19 940 748

• PATENT ABSTRACTS OF JAPAN vol. 1999, no. 10, 31. August 1999 (1999-08-31) & JP 11 127857 A (SANKYO CO LTD), 18. Mai 1999 (1999-05-18)
• RU, KUN ET AL: "Growth inhibition and antimetastatic effect of antisense poly-DNP-RNA on human breast cancer cells" ONCOLOGY RESEARCH (1999), 11(11/12), 505-512 , XP001117724
• WINCHURCH, RICHARD A. ET AL: "Effect of synthetic polyribonucleotides on EL-4 lymphoma" J. RETICULOENDOTHEL. SOC. (1974), 18(4), 244-51 , XP001117744
• KROWN S.E. ET AL: "Phase I trials of poly(I,C) complexes in advanced cancer." JOURNAL OF BIOLOGICAL RESPONSE MODIFIERS, (1985) 4/6 (640-649). CODEN: JBRMDS, XP001118036
• BRODSKY I ET AL: "Clinical studies with ampligen (mismatched double-stranded RNA)." JOURNAL OF BIOLOGICAL RESPONSE MODIFIERS, (1985 DEC) 4 (6) 669-75. , XP002218792

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft die Herstellung eines Mittels zur Behandlung von malignen Tumoren enthaltend xenogene Oligo- oder/und Polyribonukleotide. Weiterhin betrifft sie die Verwendung dieser xenogenen Oligo- oder/und Polyribonukleotide zur Herstellung von Arzneimitteln für die Behandlung von malignen Tumoren, ausgenommen maligne Hauterkrankungen.

Hintergrund der Erfindung

In der älteren DE 199 40 748 werden xenogene Oligo- oder/und Polynukleotide als Mittel zur Behandlung von Herpesviridae oder/und Hauttumoren beschrieben. Hierbei handelt es sich stets um eine topische Behandlung. Maligne Tumoren, die maligne Hauterkrankungen ausschließen, sind jedoch topisch nicht behandelbar, sodass daraus keine Hinweise auf die Behandlung normaler maligner Tumoren entnommen werden können.

Aus der japanischen Offenlegungsschrift 11127857 werden synthetische RNAs spezieller Struktur beschrieben, die eine nicht natürlich vorkommende Zusammensetzung aufweisen. Irgendein Hinweis auf xenogene RNAs lassen sich daraus nicht entnehmen.

In WO 98/05769 werden Antisense-Oligonukleotide, die zu Abschnitten des Ribonukleotidreduktasegens komplementär sind, als Inhibitoren für die Ribonukleotidreduktaseexpression beschrieben. Derartige Antisensesequenzen kommen nicht natürlich vor und haben daher keinen Bezug zu xenogenen RNAs.

Kum Ru et al. in Oncology Research, Vol. 11 (1999), S. 505-512 beschreiben ebenfalls nicht natürlich vorkommende Antisenseoligo-RNAs (poly-DNP-RNA) als Hemmstoffe für Brusttumoren. Auch hier handelt es sich wieder um nicht natürlich vorkommende synthetische Substanzen.

Richard A. Winchurch et al. beschreiben ebenfalls die Wirkungen des synthetischen Poly-RNA-Komplexes poly A=U auf ein EL-4 Lymphom. Derartige synthetische Komplexe sind völlig verschieden von xenogenen RNAs.

Susan E. Krown et al. in J. Of Biol. Response Modifiers (1985), Seite 640 ff. werden ebenfalls synthetische doppelsträngige RNA-Komplexe mit Polylysin und gegebenenfalls Carboxymethylcellulose beschrieben für die Behandlung fortgeschrittener Krebserkrankungen. Auch hier handelt es sich nicht um xenogene RNAs.

In Isadore Brodsky et al. in J. Biol. Response Modifiers wird über Versuche mit einer synthetisch hergestellten, als Ampligen bezeichneten doppelsträngigen (ds)RNA bei Metastasen berichtet. Wesentich war hierbei, dass die (ds)RNA ungepaarte Regionen aufweist. Derartige Substanzen kommen in natürlichen xenoegnen RNAs nicht vor.

In der WO99/07394 wird vorgeschlagen, einen Tumor mit einer pharmazeutischen Zusammensetzung zu behandeln, die aus einem Herpes simplex-Virus, der nur Tumorzellen infiziert, und einem für das Virus geeigneten Träger besteht. Das Herpes simplex-Virus muss dabei eine für einen Immunmodulator codierende exprimierbare Nucleotidsequenz aufweisen. Eine Behandlung von malignen Tumoren mit xenogenen Oligo- oder/und Polynukleotiden lässt sich daraus nicht entnehmen oder herleiten.

Aus der WO94/21808 ist ein Verfahren zur Herstellung von Krebsvakzinen bekannt, bei dem ein Komplex aus DNA, die für ein immunstimulierendes Polypeptid codiert mit einer DNA-bindenden Substanz, wie Polylysin, konjugiert ist. Hierbei werden Tumorzellen oder Fibroblasten kultiviert und dann ex vivo mit einer Zusammensetzung transfiziert, die ein DNA-Molekül, das für ein immunstimulierendes Polypeptid codiert, und ein Konjugat zwischen einem DNA-bindenden Molekül und einem endosomolytisch wirkenden Mittel enthält. Irgendein Hinweis auf die Verwendung xenogener Oligo- oder/und Polynukleotide findet sich dort nicht.

Ende der 60er und anfangs der 70er Jahre fand man im Rahmen der Transplantationsforschung, dass mit xenogenen heterogenen Nukleinsäuren vorbehandeltes Gewebe oder schwache Antigene in verschiedenen immunologischen Untersuchungsmethoden wesentlich erhöhte Antititer aufwiesen. Diese Ergebnisse wurden mit einer Anzahl verschiedener Antigene in *in vitro* und *in vivo* Untersuchungen weiter bestätigt. Es gab jedoch keine Hinweise, dass Nukleinsäuren und insbesondere Oligo- oder/und Polyribonukleotide xenogenen Ursprungs zur Behandlung von malignen Tumoren geeignet sein könnten.

Vor allem in den USA wurden zur gleichen Zeit Versuche mit definierten synthetischen Poly- und Oligonukleotiden, besonders Ribonukleotiden, angestellt, die aber wegen der hohen Toxiztität *in vivo* nicht weiterverfolgt wurden.

Aufgabe der vorliegenden Erfindung war daher die Herstellung eines Arzneimittels, das zur Behandlung von malignen Tumoren geeignet ist. Maligne Tumore im Sinne der vorliegenden Erfindung umfassen maligne Hauterkrankungen nicht.

Die Aufgabe wird erfindungsgemäß gelöst durch ein Verfahren zur Herstellung eines Arzneimittels für die Behandlung von malignen Tumoren, welches als Wirkstoff xenogene RNA von Organismen, die entwicklungsgeschichtlich dem zu behandelnden Organismus möglichst fernstehen, bevorzugt in Form ihrer Konjugate mit Zellen des zu behandelnden Tumors, enthält.

Xenogen bedeutet im Sinne der vorliegenden Erfindung, dass die Ribonukleinsäure aus einem anderen als dem damit zu behandelnden Organismus stammt, also solche Oligo- oder/und Polyribonukleotide, welche nicht aus demselben Organismus stammen, dem das Arzneimittel verabreicht werden soll. Bevorzugt handelt es sich bei den erfindungsgemäß verwendeten xenogenen Oligo- oder/und Polyribonukleotiden um solche aus Tiergeweben (z.B. Rindergewebe, fötales Kälbergewebe), Pflanzen und Einzellern, vorzugsweise aus Hefezellen (insbesondere Saccharomyces cerevisiae). Bei Arzneimitteln für den Menschen wird somit vorzugsweise RNA aus Tiergeweben oder besonders bevorzugt aus Pflanzen oder Einzellern, wie etwa Hefe, verwendet.

Die erfindungsgemäß verwendete RNA ist untoxisch und allein nicht antigen.

Es können Präparationen der Gesamt-RNA und deren Salze und Verbindungen wirksam eingesetzt werden. Besonders ist tRNA bevorzugt. Als Art der Gewinnung von erfindungsgemäß verwendbaren RNAs ist insbesondere die Phenolextraktion bevorzugt, speziell die hierin als Methode I und II bezeichneten Verfahren.

Weiter wurde gefunden, dass die xenogenen Ribonukleinsäuren (RNA) verbunden mit Peptiden, Polypeptiden und Proteinen deren Antigenität heben. Aufgrund dieser Beobachtungen wurde Tumorgewebe der betroffenen Patienten mit xenogener RNA sowohl *in vitro* als auch *in vivo* behandelt. Vorzugsweise erfolgt die Behandlung der Zellen des Tumorgewebes eines Patienten mit der xenogenen RNA, vorzugsweise mit tRNA, *in vitro.* Das Gemisch wird dann dem Patienten systemisch verabreicht.

Zusätzlich zur Therapierung von Menschen mit den xenogenen Oligo- oder/und Polyribonukleoitden dieser Erfindung können auch Warmblütler, wie z.B. Pferde, Rinder, Schafe etc., so behandelt werden.

Die Erfindung wird weiter durch die nachfolgenden Beispiele und Versuchsergebnisse erläutert.

**Beispiele**

**Beispiel 1**

**Gewinnung der erfindungsgemäß verwendbaren Oligo- oder/und Polyribonukleotide**

Die einschlägige Literatur beschreibt zahlreiche Methoden zur Gewinnung von Nucleinsäuren, Nukleotiden und Nukleosiden, die jedem einschlägig Erfahrenen bekannt sind. Zwei Methoden mit geringen Modifikationen, beide auf Phenolisierung beruhend, kommen hier vorzugsweise zur Anwendung, Methode I zur Gewinnung der Gesamt-RNA (Georgiev, G. P. und Mantieva, V. L., Biochim. Biophys. Acta 61, 153 (1962)) und Methode II zur Gewinnung der tRNA (Bauer, S. et al., Biotechnology and Bioengineering 15, 1081 (1973)). Beide Methoden dienen der Extraktion größerer Mengen.

Methode I

Eine 15 % Suspension von Bierhefe (Saccharomyces cerevisiae) wurde in Puffer (A) [0,001 M EDTA, 0,01 M Tris-HCl Puffer, pH 5-6, 25 % Sucrose, 0,5% SDS (Natriumdodecylsulfat), 0,3% Na-Desoxycholat] wurde in einem Waring Blendor bei 10 °C mit 3000 UPM 3 Minuten lang homogenisiert. Das Homogenat wurde mit dem gleichen Volumen der Lösung (B) [ 80% rekristallisiertes Phenol in Puffer (A), 0,1 % 8-Hydroxychinolin, 1,2 % Diethylpyrocarbonat] versetzt und dann 30 Minuten bei 60 °C langsam gerührt. Alle Pufferlösungen wurden mit deionisiertem Wasser hergestellt, das vorher mit Bentonit aufgeschüttelt worden war. Danach wurde das phenolisierte Homogenat bei Raumtemperatur, ca. 20 °C, 15 Minuten mit 10.000 g zentrifugiert. Die wässrige Phase wurde abgezogen, die Phenol- und die Zwischenphase wurden verworfen. Die wässrige Phase wurde mit dem gleichen Volumen eines 1:1 Gemisches der Lösung (B) und Chloroform-Isoamylalkohol (96:4) versetzt und wie oben beschrieben extrahiert. Die wässrige Phase wurde 3 Mal mit einem halben Volumen Diethylether ausgeschüttelt, um das retliche Phenol zu entfernen. Die Lösung wurde auf 2% Natriumacetat gebracht und die RNA mit 2,5 Volumina absoluten Ethanols ausgefällt.

Die gefällte RNA wurde bei 0°C und 5000 UPM abzentrifugiert und in einem eiskalten 0,01 M Tris-HCl Puffer, pH 7,0, und 0,001 M $MgCl_2$ aufgenommen. Zum Abbau eventueller DNA wurde die Lösung mit elektrophoretisch reiner pankreatischer DNase (4 $\mu$g/ml) versetzt und während 3 Stunden bei 22°C inkubiert. Dann wurden Proteinreste, die DNase und die RNasen mit Pronase (10 $\mu$g/ml) während 3 Stunden bei 37°C verdaut. Während dieser Zeit wurde auch die Pronase durch Selbstverdauung zerstört. Die RNA-Lösung wurde wie oben beschrieben mit der Lösung (B) 20 Minuten bei 60°C unter sanftem Rühren extrahiert, die Phasen durch Zentrifugation getrennt, die wässrige Phase abgezogen und mit Diethylether ausgeschüttelt. Nach Zusatz von Natriumacetat (Endkonzentration 2%) wurde die RNA mit 2,5 Volumina Ethanol gefällt und abzentrifugiert. Der Niederschlag wurde in kaltem 2 %-igen Natriumacetat aufgenommen, mit 2,5 Volumina Ethylalkohol gefällt und über Nacht bei -20°C im Alkoholgemisch stehen gelassen. Dann wurde das Präzipitat abzentrifugiert, zweimal mit 75 %-igem, zweimal mit absolutem Ethanol und zweimal mit Diethylether gewaschen. Nach Trocknen im Wärmeschrank wurde eine lockere, trockene RNA erhalten, die in einem dunklen Glasgefäß bei Raumtemperatur gelagert wurde.

Methode II

Diese Methode dient auch zur Extraktion großer Hefemengen (Kilogrammengen).

Ein gegebenes Gewicht Hefe wurde in der vierfachen Menge Puffer (A) (s. oben Methode I) im Kälteraum homogenisiert. Dem Homogenat wurden 40% Vol./Vol. der Phenollösung (B) und 5 % Gew./Vol. Eiswürfel aus deionisiertem Wasser zugesetzt und 30 Minuten lang gerührt. Der Überstand wurde abgesaugt und noch zweimal, wie unter Methode I beschrieben, phenolisiert. Die wässrigen Überstände wurden in einem Gefäß gesammelt, in dem sich eine DEAE-Cellulose-Aufschwemmung (ca. 10 % Gew./Vol., Whatman DE-22), entsprechend dem halben Volumen der gesammelten Überstände, befand. Die DEAE Aufschwemmung wurde 30 Minuten durch Rühren in Suspension gehalten. Dann ließ man die DEAE während einer Stunde sedimentieren. Der Überstand wurde abgesaugt. Inzwischen wurden die Zwischen- und die Phenolphase noch zweimal mit der aliquoten Menge der Lösung (C) (83 % deionisiertes Wasser, 15 % Gew./Vol. Eiswürfel, 2 % Mg-acetat-Konzentrat [0,5M Mg-acetat in 0,25 Mercaptoethanol] 30 Minuten lang gerührt und dann 70-80 Minuten separieren gelassen. Die wässrigen Überstände wurden in das Gefäß mit der DEAE übertragen, wieder gerührt und sedimentieren gelassen. Der Überstand wurde abgesaugt und die DEAE, wie oben, erst mit Lösung C zweimal, dann nochmals mit Lösung (D) (2 Volumen Mg-azetat Konzentrat, 2 Volumen NaCl-Konzentrat [3,75 M NaCl in Wasser], 0,2 Volumen Tris-HCl Konzentrat [2,5 M Tris-HCl, pH 7,5 in Wasser, 96 Volumen Wasser]) gewaschen.

Die DEAE-Cellulose wurde dann in eine Säule gepackt, die unten abgeschlossen war. Alle weiteren Schritte wurden im Kaltraum bei 4°C ausgeführt. Die Säule wurde mit der 12-fachen Menge des Säuleninhalts der Lösung (D), Flussgeschwindigkeit 1, 4 l/h, (nur durch Schwerkraft) gewaschen. Dann wurde die tRNA mit Lösung E [2 Volumen Mg-acetat-Konzentrat, 0,2 Volumen Tris-HCl-Konzentrat, 14 Volumen Na-Cl-Konzentrat, und 84 Volumen Wasser, Endkonzentration von NaCl 0,525 M, mit einem Fluss von 3 1/h eluiert. Die Fraktionen, die mehr als 35 $A_{260\,nm}$ Einheiten/ml enthielten, wurden vereint und mit 1,5 Volumen Ethanol ausgefällt. Der weitere Vorgang entsprach Methode 1.

Alternativ kann das letzte Präzipitat in Wasser aufgenommen und lyophilisiert werden.

Eine Variante dieser Methode ist die übliche Phenolisierung des Ausgangsmaterials: Aus der Oberphase wird die Roh-tRNA mit Isopropanol gefällt. Der Niederschlag wird nach der Zentrifugation mit dem Natriumacetatpuffer extrahiert und an der DEAE-Zellulose chromatographiert. Die Elution erfolgt mit dem Natriumacetat/ Natriumchlorid-Gradienten, wie sie den in der Materie bewanderten Biochemikern bekannt ist. Die geeigneten Fraktionen, siehe oben, werden mittels Quotientenmessung bestimmt und vereinigt. Die tRNA wird mit Ethanol gefällt, der Niederschlag wie oben aufgenommen und vorzugsweise lyophilisiert.

Die folgenden Tests wurden zur Untersuchung der Reinheit der Gesamt-RNA und der tRNA und zu deren Charakterisierungen angewendet:

Eiweiß wurde nach Lowry, O.H. et al. (J. Biol. Chem. 193, 265 (1951)) und durch $A_{260}/A_{280} \cong 2$, DNA nach Dische (Mikrochemie 8, 4 (1930), Gesamt-RNA nach Mejbaum (Physiol. Chem. 258, 117 (1939)), quantitative Bestimmung der tRNA und des Aminosäureneinbaus nach Sprinzl und Sternbach (Methods in Enzymology 59, 182 (1979)), Toxizität nach M. Nöldner (persönliche Mitteilung), Pyrogenfreiheit *in vitro* nach DAB 1997 (LAL-Test) und *in vivo* nach Ph.Eur. / DAB 1997 bestimmt.

Ergebnisse der Untersuchungen:

(Eigenschaften der Gesamt-RNA und der tRNA, Durchschnittswerte aus zehn Testungen)

Absorption

$$A_{260}/A_{280} \quad \cong \quad 1,94 \sim 2,0$$

C, H, N Analyse

| | | |
|---|---|---|
| C | 32,67 | 32,42 |
| H | 5,22 | 5,20 |
| N | 2,29 | 2,00 |

mit korrespondierenden Werten verschiedener Gesamt- und tRNAs.

### UV und IR Spektren

Die UV- und IR-Spektren variieren, sie sind fast gleich, aber nicht identisch, entsprechend biologischen Substanzen.

### Molekulargewicht

Gesamt- und tRNA aus Hefe $\cong$ 22000-27000 Dalton Durchschnittswert, variierend bei verschiedenen Präparationen ;

| Protein | DNA (Gesamtinhalt) |
|---|---|
| 2,3 % | neg. Gesamt-RNA Saccharomyces cerevisiae |
| 1,9 % | neg. tRNA Saccharomyces cerevisiae |
| 0,9 % | neg. Gesamt-RNA bovinen Ursprungs |

Durchschnittliche, allgemein übliche Qualität. Verbessete Reinheit brachte bei unverhältnismäßigem höheren Aufwand keine signifikant verbesserte therapeutische Wirkung.

### Aminosäureneinbau bei tRNA, Mittelwert von 10 Untersuchungen

| Lysin | 69 - 85 | pMol / $A_{260}$ Einheit |
|---|---|---|
| Phe | 41 - 55 | |
| Ser | 39 - 50 | |
| Val | 77 - 90 | |

Diese Mittelwerte ändern sich bei Hefen verschiedener Lots im angegebenen Rahmen.

### Toxizität

Prüfung auf akute Toxizität an der Maus:

| Tiere: | | NMRI Mäuse, männlich, Fa. Janvier, Frankreich |
|---|---|---|
| Applikation: | | intravenös in eine Schwanzvene |
| Beobachtungsdauer: | | 24 Stunden |
| Stichprobenumfang: | | n = 10 in der höchsten Konzentration |
| Testsubstanz: | a. | bovine Gesamt RNA |
| | b. | tRNA aus Biehefe (Saccharomyces cerevisiae) |
| Lösungsmittel: | 0,9% | NaCl in Wasser p.i. |

Ergebnis:

Bis zu einer maximalen Dosierung von 1g/kg/10ml i.v. zeigten die Versuchstiere innerhalb des Beobachtungszeitraums von 24 Stunden keinerlei Auffälligkeiten.

### Pyrogenfreiheit

A. Der Pyrogengehalt der Gesamt-RNA sowie der t-RNA, beide wie bisher beschrieben, wurde mit dem in-vitro-Test auf Endotoxine nach DAB 1997 (LAL TEST) und an Kaninchen nach Ph. Eur. /DAB 1997 bestimmt.

1. Gesamt-RNA

Endotoxin Standard EC 5
Amoebozytenlysat

- Deklarierte Empfindlichkeit:       0,06 EU/ml
- Gefundene Empfindlichkeit:       0,06 EU/ml

Prüflösung: 100mg RNA gelöst in 20ml Wasser-LAL (0,5%)
Ergebnis:

Der Endotoxingehalt der Prüflösung 0,5% 1:5 mit Wasser-LAL verdünnt: < 0,03 EU/ml.

2. tRNA
Endotoxin Standard EC 5
Amoebozytenlysat

- Deklarierte Empfindlichkeit:       0,06 EU/ml
- Gefundene Empfindlichkeit:       0,06 EU/ml

Prüflösung: 100mg RNA gelöst in 20ml Wasser-LAL (0,5%)
Ergebnis:
Der Endotoxingehalt der Prüflösung 0,5% 1:10 mit Wasser-LAL verdünnt: < 0,03 EU/ml.


B. *In vivo* Prüfung auf Pyrogenfreiheit nach DAB/Ph.Eub., Stand 2000
1. Gesamt-RNA
Prüflösung 1% der Testsubstanz in pyrogenfreiem Wasser p.i.

        Dosis:    1,0 mi/Tier
        Tiere:    3 Kaninchen, entsprechend DAB/Ph. Eub., Stand 2000

Ergebnis:
Summe der Temperaturdifferenzen von 3 Kaninchen war 1,05 °C, Pyrogene sind somit nicht nachweisbar.
2. tRNA
Prüflösung 1% der Testsubstanz in pyrogenfreiem Wasser p.i.

        Dosis:    1,0 mi/Tier
        Tiere:    2 mal 6 Kaninchen, entsprechend DAB/Ph. Eub., Stand 2000

Ergebnis:

a. Summe der Temperaturdifferenzen von 6 Kaninchen: 1,02 °C
b. Summe der Temperaturdifferenzen von 6 Kaninchen: 1,06 °C, Pyrogene nicht nachweisbar.

**Beispiel 2**


Nachweise der Tumor-Wirksamkeit der Substanzen dieser Erfindung

10 Patienten, die an diversen Karzinomen litten, operiert waren, deren Körper von zahlreichen Metastasen befallen war und die auf Chemotherapie nicht mehr ansprachen, deren Lebenszeit auf wenige Wochen von den behandelnden Onkologen beurteilt wurde, wurden mit einem tRNA/Tumorzellen-Konjugat, Inkubation ca. 30 Minuten, $6 \times 10^6$ Tumorzellen mit 50-100 mg tRNA systemisch, 2 mal innerhalb von 14 Tagen behandelt.
Eine Patientin wurde nach dieser Behandlung auch mit einem leichten Chemotherapeutikum supportiv über wenige Wochen behandelt. Nach fast 2 Jahren verstarb sie unabhängig von ihrer Grunderkrankung.
Alle anderen der behandelten Patienten überlebten > 1-2 Jahre ohne Chemotherapie bei guter Lebensqualität ohne Nebenerscheinungen.
Diese Ergebnisse rechtfertigen den Einsatz der RNA bei Patienten, besonders da sie nicht mehr auf Chemotherapie ansprachen, *in faust* waren, und keinerlei Nebenwirkungen oder toxische Erscheinungen während der verlängerten Lebenszeit zu beobachten waren.

**Patentansprüche**

1.  Verfahren zur Herstellung eines Arzneimittels für die Behandlung von malignen Tumoren, ausgenommen maligne Hauterkrankungen,
    **dadurch gekennzeichnet, dass** man xenogene RNA von Tieren, Pflanzen oder/und Einzellern, die dem zu behandelnden Organismus entwicklungsgeschichtlich möglichst fern stehen, in systemisch verabreichbare Form bringt.

2.  Verfahren nach Anspruch 1,
    **dadurch gekennzeichnet,**
    **dass** man xenogene RNA in Form ihrer Konjugate mit Zellen des zu behandelnden Tumors verwendet.

3.  Verfahren nach Anspruch 2,
    **dadurch gekennzeichnet,**
    **dass** man die xenogene RNA in in vitro mit Zellen des zu behandelnden Tumors inkubiert und das erhaltene Inkubat als Wirkstoff verwendet.

4.  Verfahren nach einem der vorhergehenden Ansprüche,
    **dadurch gekennzeichnet,**
    **dass** man physiologisch annehmbare Träger-, Hilfs-, Verdünnungsoder/und Zusatzstoffe zusetzt.

5.  Verfahren nach Anspruch 4,
    **dadurch gekennzeichnet,**
    *****dass** der Wirkstoff RNA aus Hefezellen stammt.

6.  Verfahren nach einem der vorhergehenden Ansprüche,
    **dadurch gekennzeichnet,**
    **dass** der Wirkstoff xenogene tRNA umfasst.

7.  Verfahren nach einem der vorhergehenden Ansprüche,
    **dadurch gekennzeichnet,**
    **dass** der Wirkstoff durch Phenolextraktion gewonnene xenogene RNA umfasst.

8.  Verfahren nach einem der vorhergehenden Ansprüche,
    **dadurch gekennzeichnet,**
    **dass** man das Mittel für die systemische Verabreichung in einer Dosierung von 50 bis 100 mg tRNA formuliert.

**Claims**

1.  A method for producing a medicament for the treatment of malignant tumours except malignant diseases of the skin,
    **characterized in that**
    xenogenic RNA, derived from animal tissues, plants or/and unicellular organisms which are evolutionary as far as possible distant to the organism to be treated, is brought into a systemically administerable form.

2.  The method as claimed in claim 1,
    **characterized in that**
    xenogenic RNA is used in the form of its conjugate with cells of the tumour to be treated.

3.  The method as claimed in claim 2,
    **characterized in that**
    the xenogenic RNA is incubated *in vitro* with cells of the tumour to be treated and the incubated material obtained is used as active compound.

**4.** The method as claimed in any of the preceding claims,
**characterized in that**
physiologically acceptable carriers, excipients, diluents or/and additives are added.

**5.** The method as claimed in claim 4,
**characterized in that**
the active compound RNA is derived from yeast cells.

**6.** The method as claimed in any of the preceding claims,
**characterized in that**
the active compound comprises xenogenic tRNA.

**7.** The method as claimed in any of the preceding claims,
**characterized in that**
the active compound comprises xenogenic RNA obtained by phenol extraction.

**8.** The method as claimed in any of the preceding claims,
**characterized in that**
the medicament for systemic administration is formulated as containing 50 to 100 mg tRNA.


**Revendications**

**1.** Utilisation pour la fabrication d'un médicament destiné au traitement des tumeurs malignes, à l'exception des affections cutanées malignes, **caractérisée en ce que** l'ARN xénogène provenant de tissus animeaux, de plantes et/ou des organismes unicellulaires phylogénétiquement aussi eloignés que possible de l'organisme à traiter est preparé comme administration par voie systémique.

**2.** Utilisation selon la revendication 1, **caracterisée en ce que** l'ARN xénogène est utilisé sous forme de son conjugaison avec des cellules du tumeur à traiter.

**3.** Utilisation selon la revendication 2, **caracterisée en ce que** l'ARN xénogène est incubé *in vitro* avec des cellules du tumeur à traiter et les matériaux incubés résultants sont utilisés comme substance active.

**4.** Utilisation selon l'une de la revendications précédentes, **caracterisée en ce que** des excipients, adjuvants, diluants et/ou additifs, physiologiquement acceptables, sont ajoutés.

**5.** Utilisation selon la revendication 4, **caractérisée en ce que** l'agent actif ARN provient des cellules de levure.

**6.** Utilisation selon l'une de la revendications précédentes, **caracterisée en ce que** l'agent actif comprends de l'ARN de transfert xénogène.

**7.** Utilisation selon l'une de la revendications précédentes, **caracterisée en ce que** l'agent actif contient de l'ARN par l'extraction phénolique.

**8.** Utilisation selon l'une de la revendications précédentes, **caractérisée en ce que** le dosage du medicament pour l'administration par voie systemique est de 50 à 100 mg de l'ARN de transfert.

EP 1 399 168 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19940748
- JP 11127857 B
- WO 9805769 A
- WO 9907394 A
- WO 9421808 A

**In der Beschreibung aufgeführte Nicht-Patentliteratur**

- **KUM RU et al.** *Oncology Research,* 1999, vol. 11, 505-512
- **SUSAN E. KROWN et al.** *J. Of Biol. Response Modifiers,* 1985, 640
- **GEORGIEV, G. P. ; MANTIEVA, V. L.** *Biochim. Biophys. Acta,* 1962, vol. 61, 153
- **LOWRY, O.H. et al.** *J. Biol. Chem.,* 1951, vol. 193, 265
- **DISCHE.** *Mikrochemie,* 1930, vol. 8, 4
- **MEJBAUM.** *Physiol. Chem.,* 1939, vol. 258, 117
- **SPRINZL ; STERNBACH.** *Methods in Enzymology,* 1979, vol. 59, 182